**Europäisches Patentamt**

**European Patent Office**

**Office européen des brevets**

(19)

(11) Publication number : **0 482 960 A1**

(12) # EUROPEAN PATENT APPLICATION

(21) Application number : **91309914.9**

(22) Date of filing : **28.10.91**

(51) Int. Cl.⁵ : **A61B 17/04,** A61B 17/06, A61B 17/36

(30) Priority : **26.10.90 GB 9023341**

(43) Date of publication of application :
**29.04.92 Bulletin 92/18**

(84) Designated Contracting States :
**AT BE CH DE DK ES FR GB GR IT LI LU NL SE**

(71) Applicant : **RICHARDSON, Philip**
**The Bungalow Pibwrlwyd Lane Camarthen**
**Dyfed SA31 2NH (GB)**
(71) Applicant : **RICHARDSON, Margaret Pamela**
**The Bungalow Pibwrlwyd Lane Camarthen**
**Dyfed SA31 2NH (GB)**

(72) Inventor : **RICHARDSON, Philip**
**The Bungalow Pibwrlwyd Lane Camarthen**
**Dyfed SA31 2NH (GB)**
Inventor : **RICHARDSON, Margaret Pamela**
**The Bungalow Pibwrlwyd Lane Camarthen**
**Dyfed SA31 2NH (GB)**

(74) Representative : **Austin, Hedley William et al**
**Urquhart-Dykes & Lord Alexandra House 1**
**Alexandra Road**
**Swansea West Glamorgan SA1 5ED (GB)**

(54) **Surgical apparatus for suture insertion.**

(57)   The apparatus comprises suture thread (4) ; an optical fibre or fibres (2,6) for transmission of laser radiation (7) along the interior of the suture thread (4) ; and a laser source (1b) for supply of laser radiation (7) via the optical fibre or fibres (2,6) to the leading end of the suture thread (4) such that the radiation (7) can penetrate tissue (8) of the patient ; in which the leading end of the suture thread (4) can be manipulated. The suture thread (4) is inserted into tissue (8) of a patient by directing laser radiation (7) at the tissue surface so as to form a pilot hole in the tissue (8) followed by the insertion of the suture thread (4) into the pilot hole.

FIG. 2a

EP 0 482 960 A1

The present invention is concerned with an apparatus and method for insertion of suture thread into tissue of a patient.

A common procedure in medicine and surgery is the insertion of sutures into tissue; for example, in surgery a wound is closed by the application of a suture thread using a sharp needle attached to the suture thread. It is often necessary to pass the needle through tough tissue and the needle must therefore be rigid and sharply pointed. Even so, the medical operative often has difficulty in forcing the needle to satisfactorily penetrate the tissue. The application of force often results in tearing, bleeding and trauma for the patient.

The forceful use of a sharp needle also sometimes results in needle stick injury to medical personnel. Needle stick injury has been cited as a probable cause for transmission of disease from medical personnel to patient and vice-versa.

The use of lasers is well known in medicine for certain procedures e.g. in surgery, where its use is in the manner of a scalpel or probe to cut or destroy tissue or to cauterise vascular tissue. Optical fibre technology is also well understood in medicine where its use in endoscopy has become routine.

A method and apparatus has now been developed which can facilitate penetration of suture thread into tissue without the use of a needle by using laser energy to create a pilot hole in the tissue of the patient.

According to the present invention there is provided surgical apparatus comprising:

a) suture thread;

b) means for transmission of laser radiation along the interior of said suture thread;

c) a laser source for supply of laser radiation via said transmission means to the leading end of said suture thread such that said radiation can penetrate tissue of the patient; and

d) means for permitting manipulation of said leading end of said suture thread.

The present invention further comprises a method of insertion of a suture thread into tissue of a patient, which comprises directing laser radiation at the tissue surface so as to form a pilot hole in said tissue followed by the insertion of said suture thread into the pilot hole. The method according to the invention is preferably carried out using apparatus as just described.

The laser source is typically of the YAG (yttrium aluminium garnet) or argon type, which have wavelengths in the near infra-red range or (with appropriate waveguides) of the carbon dioxide type.

The means for transmission of laser radiation is typically an optical fibre or fibres or similar waveguide.

It is preferred that such an optical fibre terminates some distance from the tip of the suture thread in contact with the tissue in order to form a small air gap between the end of the optical fibre and the tissue. This is done to keep the end of the optical fibre clean and clear of tissue and thus avoid overheating of the end of the optical fibre.

It is further preferred that the optical fibre or fibres be contained within the suture and clad with a flexible cladding so as to provide an optical fibre capable of total internal reflection.

It is further preferred that the optical fibre or fibres be sheathed with a supple and flexible sheathing of, for example, polybutyrolactone, or a material known an polybutester (which is commercially available under the trade mark "Novafil") to form a suture thread with the necessary soft and supple handling qualities

It is further preferred that the optical fibre within the suture thread be defocused at its terminal point distal from the laser source so as to render the laser beam harmless a short distance in front of the suture thread, for example, by the provision of a convex surface at the end of the optical fibre. This is intended to protect medical personnel and patient from accidental exposure to focussed laser energy.

It is further preferred that the sheathing of the suture thread be made stiffer, typically by the application of a relatively more rigid material, for example, polypropylene, over a convenient length of the leading end of suture thread so as to enable the end of the suture thread to be manipulated through the pilot hole formed in the tissue, and to protect the optical fibre from instrument damage during manipulation.

In use, the stiffened end of the suture is applied to the tissue to be penetrated in the usual way. The laser source is activated under the control of the medical operative, and the laser energy, which may be in the infra-red band, passes at high intensity through the optical fibre within the suture thread. The laser energy, which may be defocussed by a convex surface (or other similar means) at the end of the optical fibre, and thus causes vaporisation over a relatively small area of tissue a short distance in front of the stiffened end of the suture thread.

The effect is to create a pilot hole in the tissue into which the stiffened end of the suture thread is easily inserted. A secondary effect of the cauterisation of the sides of the pilot hole by the laser leading to reduced bleeding, tearing and trauma.

In a further embodiment, the suture thread may be made one-use disposable by providing a non-disposable section of optical fibre or other delivery system from the laser source to a connecting terminal which can be clipped to the operating apron.

The disposable one-use section of the suture thread will be plugged into the connecting terminal before use.

In a further embodiment, normal light of visible frequency may be mixed with the laser infra-red light to enable the medical operative to visually detect the position of the stiffened end of the suture thread near the penetration point, where the stiffened end of the suture thread is about to emerge from the tissue.

In a further variation, visible light entering the stiffened end of the suture thread may be tested for and detected within the laser source. An electronic interlock may be provided to prevent the laser being operated when visible light is detected entering the optical fibre via the stiffened end of the suture thread. The effect is to prevent the laser being operated when the stiffened end of the suture thread is not touching or buried in tissue. To prevent damage to the optical detection system, the use of a pulsed laser is desirable, with the interlock made inoperative and thus protected whilst the laser pulse is operative. To provide additional safety in operation and to enable the medical operative to have feedback, an audible indication of laser use may be provided.

An alternative or additional feedback mechanism may be supplied by the provision of a transparent sheathing (of, for example, polybutyrolactone) to the suture thread through which visible light may be passed from the laser source. The visible light would be pulsed in direct proportion to the laser energy passing through the fibre optic core and this provide the medical operative with visual feedback of laser strength in use even when viewed using peripheral vision.

In a further embodiment of the invention, a pulsed non-laser white light may be injected into the optical fibre between the pulses of laser radiation. The visible light after travelling down the optical fibre will emerge at the stiffened end of the suture thread and will illuminate the small section of "pilot" hole formed by the previous laser pulse.

Reflected light from the surface of the "pilot" hole may travel in the reverse direction through the optical fibre and it may be possible to detect its frequency at the laser source end of the optical fibre. The purpose of this arrangement is to detect the reddish light given by passage of the stiffened end of the suture thread through normal blood-bearing tissue e.g. muscle fibre, and to differentiate in particular white fluid or tissue. This may be of importance in certain procedures involving medical apparatus such as spinal taps where there is a risk of insertion so far into the spine that the spinal cord which is primarily white tissue is damaged. The use of such a sensory arrangement interlocked with the laser source may reduce the risk.

The invention will now be further described, by way of example only, with reference to the accompanying drawings, in which like parts are denoted by like numerals and:

Figure 1 is a schematic representation of an exemplary embodiment of apparatus according to the present invention;

Figure 2a is a longitudinal sectional view of the suture thread used in apparatus according to the invention when passing through tissue; and

Figure 2b is a cross-sectional view of the suture thread illustrated in Figure 2a, along plane A-A.

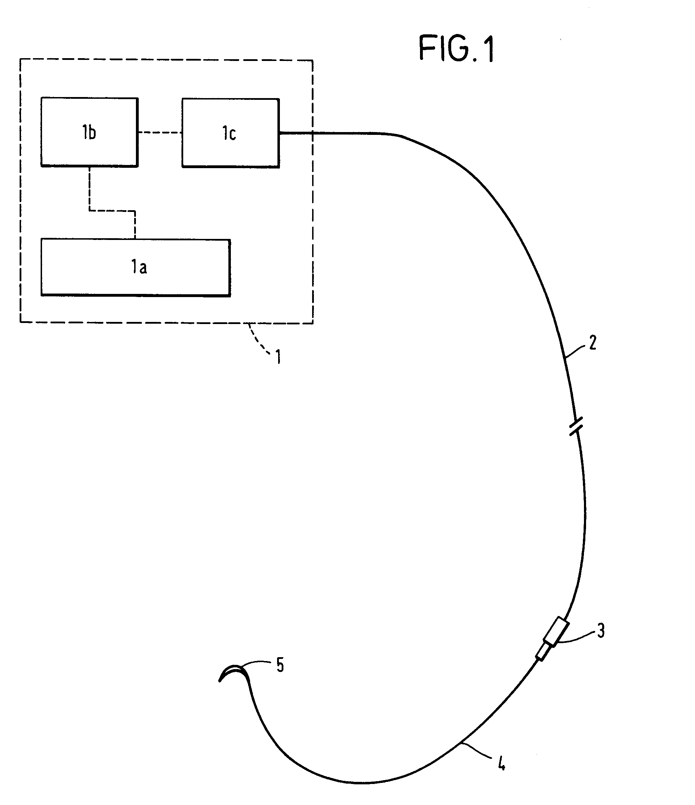

Referring to Figure 1, there is shown apparatus comprising a laser unit further comprising a power supply 1a, laser source 1b and coupling optics 1c, connected to a non-disposable section of clad optical fibre or delivery system 2. A connecting terminal 3 joins delivery system 2 to a one-use disposable suture thread 4. Suture thread 4 comprises an optical fibre surrounded by a flexible cladding and sheathing, and is plugged into connecting terminal 3 before use. At the leading end of suture thread 4, the sheathing 5 is stiffened to facilitate manipulation of suture thread 4 through tissue.

Referring to Figure 2a, there is shown suture thread 4 comprising an optical fibre 6 along which laser radiation passes from laser unit 1. Defocussed laser radiation 7 emitted by optical fibre 6 can only penetrate through a short distance of tissue 8. Optical fibre 6 is contained within flexible cladding 9 to ensure total internal reflection within optical fibre 6, cladding 9 itself being further surrounded by sheathing 5 to provide the required handling properties of suture thread 4. Optical fibre 6 is separated from tissue 8 by an air gap 10 to protect optical fibre 6 from damage during use. As suture thread 4 passes through tissue 8 the sides 11 of the resulting pilot hole are cauterised by defocussed laser radiation 7.

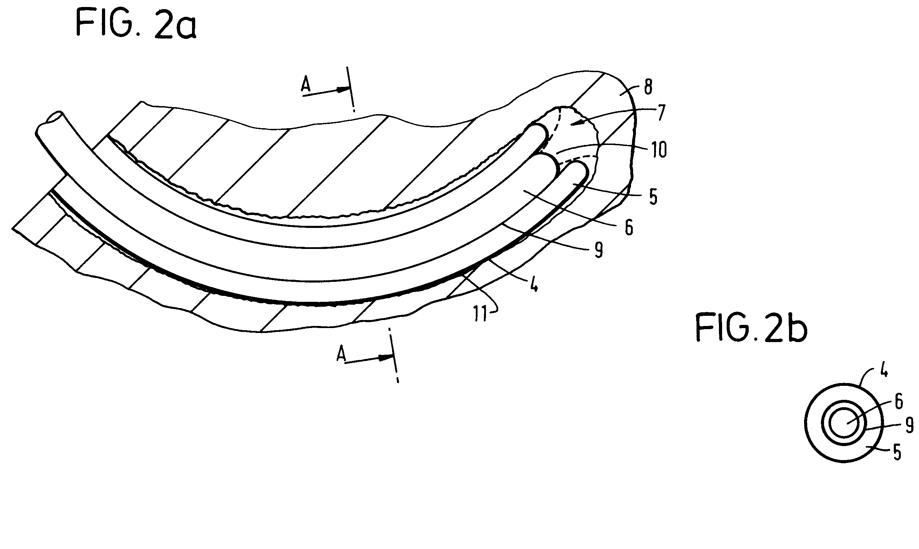

Figure 2b shows the suture thread comprising optical fibre 6 surrounded by cladding 9 and sheathing 5.

## Claims

1. Surgical apparatus comprising:
   a) suture thread;
   b) means for transmission of laser radiation along the interior of said suture thread;
   c) a laser source for supply of said laser radiation via said transmission means to the leading end of said suture thread such that said radiation can penetrate tissue of the patient; and
   d) means for permitting manipulation of said leading end of said suture thread.

2. Apparatus according to claim 1, wherein said laser radiation has a wavelength in the near infrared range, preferably mixed with visible light.

3. Apparatus according to claim 1 or 2, wherein said transmission means comprises at least one optical fibre, which preferably terminates some distance from the tissue-conucting tip of said suture thread so as to form a small air gap between the end of said optical fibre and said tissue.

4. Apparatus according to claim 3, wherein said optical fibre is contained within said suture thread and clad with a flexible cladding so as to provide an optical fibre capable of total internal reflection.

5. Apparatus according to any of claim 3 or 4, wherein said optical fibre is sheathed with a flexible sheathing, of, for example, polybutyrolactone.

6. Apparatus according to any of claims 3 to 5, wherein said optical fibre within said suture thread is defocused at the terminal point of said fibre distal from said laser source.

7. Apparatus according to any of claims 1 to 6, wherein the sheathing of said suture thread is stiffened by the provision thereon of a relatively more rigid material such as polypropylene.

8. Apparatus according to any of claims 3 to 7, wherein said apparatus comprises a non-disposable section of optical fibre leading from said laser source to a connecting terminal, said apparatus being preferably provided in use with a one-use section of said suture thread arranged to be plugged into said connecting terminal.

9. Apparatus according to any of claims 1 to 8, which is provided with an electronic interlock to prevent said laser being operated when visible light is detected entering said optical fibre via said leading end, the laser radiation source being preferably provided with means for pulsing said radiation.

10. Apparatus according to any of claims 1 to 9, wherein a transparent sheathing is provided on said suture thread through which visible light can be passed from said laser source.

FIG.1

FIG. 2a

FIG.2b

**European Patent Office**

## EUROPEAN SEARCH REPORT

Application Number

EP 91 30 9914

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-8 704 080 (LONGMORE) <br> * Claims 6,7; figures * <br> --- | 1 | A 61 B 17/04 <br> A 61 B 17/06 <br> A 61 B 17/36 |
| A | SOVIET INVENTIONS ILLUSTRATED, section PQ, week 8634, 5th September 1986, class P31, AN 86-223672/34, Derwent Publications Ltd, London, GB; & SU-A-1022 355 (SKOBELKIN) 07-02-1986 <br> * Abstract; figure * <br> --- | 1 | |
| A | US-A-4 830 462 (KARNY) <br> * Column 1, lines 6-17; abstract; figures * <br> --- | 2-5 | |
| A | US-A-3 538 919 (MEYER) <br> * Abstract; figures * <br> --- | 3,9 | |
| A | US-A-2 182 565 (MICRA) <br> ----- | | |
| | | | TECHNICAL FIELDS SEARCHED (Int. Cl.5) |
| | | | A 61 B |

The present search report has been drawn up for all claims

| Place of search | Date of completion of the search | Examiner |
|---|---|---|
| THE HAGUE | 19-12-1991 | KLEIN C. |

EPO FORM 1503 03.82 (P0401)